# EUROPÄISCHE PATENTANMELDUNG

(11) **EP 3 885 387 A1**
(43) Veröffentlichungstag der Anmeldung: **29.09.2021**
(21) Anmeldenummer: 20165485.2
(22) Anmeldetag: 25.03.2020
(51) Int. Cl.: C08G 18/80, C07D 263/10, C08G 18/73, C08G 18/78, C08G 18/79, C09D 175/00

(54) **POLYISOCYANAT-BASIERTE POLYADDITIONSVERBINDUNGEN MIT FÜNFGLIEDRIGEN CYCLISCHEN IMINOETHER-STRUKTURELEMENTEN**

(71) Anmelder: Covestro Deutschland AG, 51373 Leverkusen (DE)
(72) Erfinder: BEUCK, Saskia, 51375 Leverkusen (DE); KUTZ, Anne, 51399 Burscheid (DE); LAAS, Hans-Josef, 51519 Odenthal (DE); FLUEGEL, Magdalena, 51145 Köln (DE); DORNBUSCH, Michael, 40591 Düsseldorf (DE); KNOSPE, Philipp, 46459 Rees (DE); BOEHM, Patrick, 47805 Krefeld (DE)
(74) Vertreter: Levpat

(57) **Zusammenfassung**

Die vorliegende Erfindung betrifft neue Polyisocyanat-basierte Polyadditionsverbindungen mit fünfgliedrigen cyclischen Iminoether-Strukturelementen, ein Verfahren zur Herstellung dieser Polyadditionsverbindungen sowie die Verwendung dieser Verbindungen zur Herstellung von Polyurethankunststoffen. Außerdem betrifft die Erfindung Beschichtungsmittel enthaltend die Polyisocyanat-basierten Polyadditionsverbindungen sowie entsprechend beschichtete Substrate.

## Beschreibung

Die vorliegende Erfindung betrifft neue Polyisocyanat-basierte Polyadditionsverbindungen mit fünfgliedrigen cyclischen Iminoether-Strukturelementen, ein Verfahren zur Herstellung dieser Polyadditionsverbindungen sowie die Verwendung dieser Verbindungen zur Herstellung von Polyurethankunststoffen. Außerdem betrifft die Erfindung Beschichtungsmittel enthaltend die Polyisocyanat-basierten Polyadditionsverbindungen sowie entsprechend beschichtete Substrate.

Fünfgliedrige cyclische Iminoether sind als Oxazoline bekannt und wurden in der Vergangenheit als Ausgangsstoffe für Kunststoffe, Harze und Klebstoffe verwendet. Die Herstellung von Polyiminoethern wird in verschiedenen Quellen beschrieben.

Die Vernetzung von monomeren 2-Oxazolinen sowie die Herstellung von Oxazolin-basierten Polymeren sind Literatur-bekannt. Durch Einsatz von geeigneten sauren Initiatoren gelingt beispielsweise die Selbstpolymerisation von monomeren 2-Alkyl-substituierten Oxazolinen. Die lebende kationische Polymerisation von 2-Oxazolinen führt zur Bildung von Poly(2-oxazolin) mit Polyamid-Einheiten, ein Plattform-Polymer kommerzialisiert durch beispielsweise Ultroxa®. Durch Variation des Substituenten in Position 2 können die Eigenschaften und damit verbundenen Anwendungsgebiete des Oxazolin-basierten Polymers beeinflusst werden.

Unter dem Namen EPOCROS wurden Oxazolin-funktionelle reaktive Co-Polymere von Nippon Shokubai Co. Ltd. basierend auf einer Technologie von DOW Chemical Company USA kommerzialisiert. Anwendung finden diese Polymere beispielsweise als wasserbasierte Vernetzer. Die Umsetzung von 2-Oxazolinen mit Carboxyl-Gruppen führt durch Ringöffnung zur Ausbildung stabiler Amidbindungen, ähnlich der Strukturen entstehend durch Selbstpolymerisation. Reaktivität der Oxazolin-Gruppen findet sich auch gegenüber Epoxy-Gruppen sowie gegenüber Thiolen und Phenolen.

In allen Fällen führt die Selbstpolymerisation durch Ringöffnung zu vernetzten Strukturen mit einem hohen Anteil an tertiären Amidbindungen, ähnlich Polypeptiden. Die Weiterreaktion der erhaltenen Poly(2-oxazoline) mit Isocyanaten wird von Jordan et al. (Macromol. Chem. Phys. 2006, 207, 183) beschrieben.

Die Herstellung von Polyadditionsverbindungen mit Oxazolin-Strukturelementen ist von geringerer Bekanntheit und wurde in folgenden Publikationen beschrieben.

EP 0 472 560 beschreibt cyclische Polyiminoether, entstanden aus der Umsetzung von Iminoethern mit Diisocyanaten. Es werden wachsartige, hochviskose Substanzen erhalten. Als geeignete Iminoether werden hier fünf oder sechsgliedrige cyclische Verbindungen mit Isocyanat-reaktiven Substituenten in Position 2 verwendet.

Gegenstand der DE 44 42 908 sind Oxazolin-terminierte Polyurethane auf der Basis von Polyurethan-Prepolymeren, hergestellt aus Diisocyanaten und Polyolen, und hydroxyl-funktionellen Iminoethern, bekannt aus EP 0 472 560. In diesem Verfahren erfolgt die Herstellung des hochviskosen Oxazolin-terminierten Polyurethans in einer zwei-stufigen Synthese. Weiterhin wird die Verwendung der hergestellten Verbindung als in der Hitze härtbares 1K-System zur Verwendung in Beschichtungen, Klebstoffen und Dichtungen beschrieben.

US 5240744 beschreibt ein Verfahren zur Herstellung von Beschichtungsmitteln auf Basis von cyclischen Polyiminoethern. In der Anwendung favorisiert sind difunktionelle cyclische Polyiminoether, die aus Polyisocyanaten und hydroxyl-funktionellen Monoiminoethern hergestellt werden. Weiterhin enthalten die Beschichtungsmittel verschiedene Additive, Monoiminoether und/oder Lactone und werden über Polymerisationskatalysatoren in der Hitze vernetzt.

Nach DE 41 04 789 werden durch Umsetzung von Diisocyanaten mit Monoiminoethern cyclische Polyiminoether mit Amid-Struktureinheiten für die Verwendung in Beschichtungsmitteln, Klebemassen oder Gießmassen erhalten.

Gegenstand der US 4912154 sind wasserverdünnbare Säure-funktionelle Polyurethane, hergestellt aus substituierten Oxazolinen und Diisocyanaten, sowie einer gegenüber Isocyanat-reaktiven, Säure-funktionellen Verbindung. Die beschriebenen Oxazoline zeigen Fettsäure- sowie Hydroxy-Substitution auf. Weiterhin wird die Verwendung des Polymers in wässriger Dispersion für Beschichtungsmittel beschrieben.

US 6306967 beschreibt feste Oxazolin-terminierte, Urethan-funktionelle Polyadditionsverbindungen auf Basis von difunktionellen Polyisocyanaten und Hydroxy- oder Amin-funktionellen Oxazolin-Derivaten. Auch hier werden Oxazolin-Derivate verwendet, die an Position 2 mit einer funktionellen Gruppe substituiert sind. Die beschriebenen Verbindungen werden im Bereich der Pulverlacke eingesetzt.

Die bekannte Literatur fokussiert sich vorwiegend auf die Umsetzung von Isocyanat-Verbindungen mit Oxazolin-Derivaten, welche Isocyanat-reaktive Gruppen in Position 2 aufweisen. Als nachteilig wird hier die aufwändige und kostenintensive Herstellung der Oxazolin-Derivate, sowie die geringe strukturelle Vielfalt der verfügbaren Oxazoline zur Herstellung von Polyadditionsverbindungen, welche sich auch im eingeschränkten Anwendungsbereich wieder spiegelt, gesehen.

Aufgabe der vorliegenden Erfindung war es daher neue Polyadditionsverbindungen auf Basis von Isocyanaten und kostengünstig verfügbaren Oxazolin-Derivaten mit breiten Möglichkeiten der strukturellen Vielfalt herzustellen.

Diese Aufgabe konnte überraschenderweise über die Bereitstellung von Hydroxymethyl-Derivaten der Oxazoline, welche Isocyanat-reaktive Gruppen in Position 4 aufweisen, gelöst werden.

Gegenstand der vorliegenden Erfindung sind Polyadditionsverbindungen, welche mindestens ein Strukturelement der allgemeinen Formel (1) 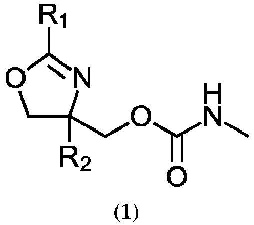 enthalten, in welcher die Reste R₁ und R₂ für gleiche oder verschiedene Reste stehen und gegenüber Isocyanatgruppen inerte, lineare oder verzweigte aliphatische, cycloaliphatische, gegebenenfalls substituierte aromatische oder araliphatische Reste mit jeweils bis zu 18 Kohlenstoffatomen stehen, die bis zu drei oder vier Heteroatome enthalten können und in Kombination untereinander zusammen einen Ring bilden können.

Gegenstand der Erfindung ist auch ein Verfahren zur Herstellung dieser Polyadditionsverbindungen durch Umsetzung einer Oxazolinkomponente A mit einer Isocyanatkomponente B, sowie die Verwendung dieser Polyadditionsverbindungen zur Herstellung von Polyurethankunststoffen insbesondere von Beschichtungsmitteln für verschiedene Substrate.

Die erfindungsgemäßen Polyadditionsverbindungen werden hergestellt durch Umsetzung einer Oxazolinkomponente A mit einer Isocyanatkomponente B.

Geeignete Oxazolinkomponente A sind beliebige OH-funktionelle 2-Oxazoline der allgemeinen Formel (2), 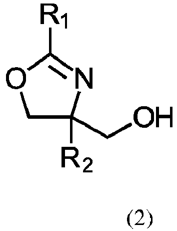 in welcher R₁ und R₂ die oben für Formel 1 beschriebene Bedeutung haben.

Aliphatische oder araliphatische Reste R₁ und R₂ sind in Formel 1 bzw. 2 beispielsweise solche mit 1 bis 18 Kohlenstoffatome, wie z. B. ein Methyl-, Ethyl-, Propyl-, Isopropyl-, n-Butyl-, sec-Butyl-, tert.-Butyl-, Pentyl-, Hexyl-, Heptyl-, Octyl-, 2-Ethylhexyl-, 2,4,4-Trimethylpentyl-, Decyl-, Dodecyl-, Tetradecyl-, Hexadecyl-, Octadecyl-, 1,1-Dimethylpropyl-, 1,1-Dimethylbutyl-, 1,1,3,3-Tetramethylbutyl-, Benzyl-, 1-Phenylethyl-, 2-Phenylethyl-, □,□ Dimethylbenzyl-, Benzhydryl-, p-Tolylmethyl-, 1-(p-Butylphenyl)-ethyl-, p-Chlorbenzyl-, 2,4-Dichlorbenzyl-, p-Methoxybenzyl-, m-Ethoxybenzyl-, 2-Cyanoethyl-, 2-Cyanopropyl-, 2-Methoxycarbonethyl-, 2-Ethoxycarbonylethyl-, 2-Butoxycarbonylpropyl-, 1,2-Di(methoxycarbonyl)ethyl-, 2-Methoxyethyl-, 2-Ethoxyethyl-, 2-Butoxyethyl-, Diethoxymethyl-, Diethoxyethyl-, 1,3-Dioxolan-2-yl-, 1,3-Dioxan-2-yl-, 2-Methyl-1,3-dioxolan-2-yl-, 4-Methyl-1,3-dioxolan-2-yl-, 2-lsopropoxyethyl-, 2-Butoxypropyl-, 2-Octyloxyethyl-, Chlormethyl-, 2-Chlorethyl-, Trichlormethyl-, Trifluormethyl-, 1,1-Dimethyl-2-chlorethyl-, 2-Methoxyisopropyl-, Butylthiomethyl-, 2-Dodecylthioethyl-, 2-Phenylthioethyl-, 2,2,2-Trifluorethyl-, 2-Phenoxyethyl-, 2-Phenoxypropyl-, 3-Phenoxypropyl-, 4-Phenoxybutyl-, 6-Phenoxyhexyl-, 2-Methoxyethyl-, 2-Methoxypropyl-, 3-Methoxypropyl-, 4-Methoxybutyl-, 6-Methoxyhexyl-, 2-Ethoxypropyl-, 3-Ethoxypropyl-, 4-Ethoxybutyl- oder 6-Ethoxyhexylrest.

Cycloaliphatische Reste R₁ und R₂ sind in Formel 1 bzw. 2 beispielsweise solche mit 5 bis 12 Kohlenstoffatome, wie z. B. ein Cyclopentyl-, Cyclohexyl-, Cyclooctyl-, Cyclododecyl-, Methylcyclopentyl-, Dimethylcyclopentyl-, Methylcyclohexyl-, Dimethylcyclohexyl-, Diethylcyclohexyl-, Butylcyclohexyl-, Methoxycyclohexyl-, Dimethoxycyclohexyl-, Diethoxycyclohexyl-, Butylthiocyclohexyl-, Chlorcyclohexyl-, Dichlorcyclohexyl-, Dichlorcyclopentylrest sowie gesättigte oder ungesättigte bicyclische Systeme wie z. B. ein Norbornyl- oder ein Norbornenylrest.

Aromatische Reste R₁ und R₂ sind in Formel 1 bzw. 2 beispielsweise solche mit 6 bis 12 Kohlenstoffatome, wie z. B. ein Phenyl-, Tolyl-, Xylyl-, o-Naphthyl-, β-Naphthyl-, 4-Diphenylyl-, Chlorphenyl-, Dichlorphenyl-, Trichlorphenyl-, Difluorphenyl-, Methylphenyl-, Dimethylphenyl-, Trimethylphenyl-, Ethylphenyl-, Diethylphenyl-, Isopropylphenyl-, tert.-Butylphenyl-, Dodecylphenyl-, Methoxyphenyl-, Dimethoxyphenyl-, Ethoxyphenyl-, Hexyloxyphenyl-, Methylnaphthyl-, Isopropylnaphthyl-, Chlornaphthyl-, Ethoxynaphthyl-, 2,6-Dimethylphenyl-, 2,4,6-Trimethylphenyl-, 2,6-Dimethoxyphenyl-, 2,6-Dichlorphenyl-, 4-Bromphenyl-, 2- oder 4-Nitrophenyl-, 2,4- oder 2,6-Dinitrophenyl-, 4-Dimethylaminophenyl-, 4-Acetylphenyl-, Methoxyethylphenyl- oder Ethoxymethylphenylrest.

Vorzugsweise stehen die Reste R₁ und R₂ in der allgemeinen Formel 1 bzw. 2 für gleiche oder verschiedene, gegenüber Isocyanatgruppen inerte, lineare oder verzweigte aliphatische oder cycloaliphatische Reste mit bis zu 18 Kohlenstoffatomen.

Besonders bevorzugt stehen R₁ und R₂ für gleiche oder verschiedene, gegenüber Isocyanatgruppen inerte, lineare aliphatische Reste mit bis zu drei Kohlenstoffatomen. Ganz besonders bevorzugt steht R₁ und R₂ jeweils für eine CH₃-Gruppe.

Derartige Ausgangsverbindungen sind bekannt und lassen sich beispielsweise durch Umsetzung von Aminoalkoholen und Säuren oder Anhydriden in einer Zyklisierungsreaktion gemäß der folgenden allgemeinen Reaktionsgleichung herstellen (Bull. Soc. Chem. Belg.,1956, Vol 65, Issue 3-4, Page 377-402):

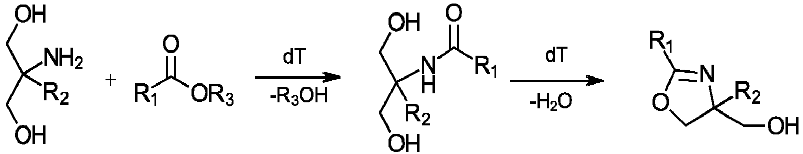

Bei der Herstellung der bevorzugt eingesetzten Oxazolinkomponente A nach dieser allgemeinen Reaktionsgleichung steht R₃ vorzugsweise für Wasserstoff. Besonders bevorzugt erfolgt die Herstellung der Oxazolinkomponente A unter Verwendung von ββ'-Dihydroxy-t-butylamin und Essigsäure zu 2,4-Dimethyl-4-hydroxymethyl-2-oxazolin.

Geeignete Isocyanatkomponenten B sind beliebige Diisocyanate, welche über Phosgenierung in der Flüssig- oder Gasphase oder auf phosgenfreiem Wege zugänglich sind. Bevorzugte Diisocyanate sind solche des Molekulargewichtsbereichs 140 bis 400 g/mol mit aliphatisch, cycloaliphatisch, araliphatisch und/oder aromatisch gebundenen Isocyanatgruppen, wie z. B. 1,4-Diisocyanatobutan, 1,5-Diisocyanatopentan (Pentamethylendiisocyanat, PDI), 1,6-Diisocyanatohexan (Hexamethylendiisocyanat, HDI), 2-Methyl-1,5-diisocyanatopentan, 1,5-Diisocyanato-2,2-dimethylpentan, 2,2,4- bzw. 2,4,4-Trimethyl-1,6-diisocyanatohexan, 1,10-Diisocyanatodecan, 1,3-und 1,4-Diisocyanatocyclohexan, 1,3- und 1,4-Bis-(isocyanatomethyl)cyclohexan, 1-Isocyanato-3,3,5-trimethyl-5-isocyanatomethylcyclohexan (Isophorondiisocyanat, IPDI), 4,4'-Diisocyanatodicyclohexylmethan, 1-Isocyanato-1 -methyl-4(3)isocyanato-methylcyclohexan, Bis-(isocyanatomethyl)-norbornan, 1,3- und 1,4-Bis(isocyanatomethyl)benzol (Xylylendiisocyanat, XDI), 1,3- und 1,4-Bis-(2-isocyanato-prop-2-yl)-benzol (Tetramethylxylylendiisocyanat , TMXDI), 2,4- und 2,6-Diisocyanatotoluol (Toluylendiisocyanat, TDI), 2,4'- und 4,4'-Diisocyanatodiphenylmethan (MDI), 1,5-Diisocyanatonaphthalin oder Mischungen aus mindestens zwei solcher Diisocyanate.

Weiterhin geeignete Isocyanatkomponenten B sind beliebige, durch Modifizierung aliphatischer, cycloaliphatischer, araliphatischer und/oder aromatischer Diisocyanate zugängliche Di- und Polyisocyanate mit Uretdion-, Isocyanurat-, Allophanat-, Biuret-, Iminooxadiazindion- und/oder Oxadiazintrionstruktur, wie sie beispielsweise in J. Prakt. Chem. 336 (1994) 185 - 200, in DE-A 1 670 666, DE-A 1 954 093, DE-A 2 414 413, DE-A 2 452 532, DE-A 2 641 380, DE-A 3 700 209, DE-A 3 900 053 und DE-A 3 928 503 oder in EP-A 0 336 205, EP-A 0 339 396 und EP-A 0 798 299 sowie in DE-A 870 400, DE-A 953 012, DE-A 1 090 196, EP-A 0 546 399, CN 105218780, CN 103881050, CN 101717571, US 3 183 112, EP-A 0 416 338, EP-A 0 751 163, EP-A 1 378 529, EP-A 1 378 530, EP-A 2 174 967, JP 63260915 und JP 56059828 beispielhaft beschrieben sind, Mischungen aus mindestens zwei solcher Di- und/oder Polyisocyanate.

Weiterhin geeignete Isocyanatkomponenten B sind beliebige Prepolymere, welche durch Umsetzung von beliebigen Diisocyanaten und/oder beliebigen Polyisocyanaten mit beliebigen Polyolkomponenten zugänglich sind. Dazu zählen auch hydrophil modifizierte Prepolymere und Di -/Polyisocyanate.

Bei der Herstellung genannter Di- und/oder Polyisocyanate und Prepolymere schließt sich an die eigentliche Modifizierungsreaktion in der Regel ein weiterer Verfahrenschritt zur Abtrennung der nicht umgesetzten überschüssigen monomeren Diisocyanate an. Diese Monomerenabtrennung erfolgt nach an sich bekannten Verfahren vorzugsweise durch Dünnschichtdestillation im Hochvakuum oder durch Extraktion mit geeigneten gegenüber Isocyanatgruppen inerten Lösemitteln, beispielsweise aliphatischen oder cycloaliphatischen Kohlenwasserstoffen wie Pentan, Hexan, Heptan, Cyclopentan oder Cyclohexan.

Bevorzugt kommen beim erfindungsgemäßen Verfahren als Isocyanatkomponenten B Diisocyanate, Polyisocyanate und Prepolymere der genannten Art zum Einsatz, die einen Gehalt an monomeren Diisocyanaten von weniger als 1 Gew.-%, vorzugsweise weniger als 0,5 Gew.-%, besonders bevorzugt von weniger als 0,3 Gew.-% aufweisen. Die Rest-Monomeren Gehalte werden nach DIN EN ISO 10283:2007-11 gaschromatographisch mit internem Standard gemessen.

Besonders bevorzugte Isocyanatkomponenten B für das erfindungsgemäße Verfahren sind solche der genannten Art mit ausschließlich aliphatisch und/oder cycloaliphatisch gebundenen Isocyanatgruppen.

Ganz besonders bevorzugt als Isocyanatkomponenten B sind Di- und/oder Polyisocyanate mit ausschließlich aliphatisch und/oder cycloaliphatisch gebundenen Isocyanatgruppen und mit Isocyanurat- und/oder Allophanatstruktur, am meisten bevorzugt solche auf Basis von PDI, HDI, IPDI und/oder 4,4'-Diisocyanatodicyclohexylmethan.

Weiterhin geeignet sind Di- und/oder Polyisocyanate und Prepolymere, welche hydrophil modifiziert sein können.

Die Herstellung der erfindungsgemäßen Polyadditionsverbindung erfolgt durch die Umsetzung der Oxazolinkomponenten A mit Isocyanatkomponenten B bei Temperaturen von 20 bis 120 °C, vorzugsweise bei 30 bis 100 °C, besonders bevorzugt bei 50 bis 90 °C unter Einhaltung eines Äquivalent-Verhältnisses von NCO-Gruppen zu NCO-reaktiven Gruppen von 1:1 bis 1:1,2. Die Umsetzung erfolgt bis zu einem NCO-Gehalt von ≤ 0,1 Gew.-%, wobei der Verlauf der Umsetzung durch Bestimmung des NCO-Gehaltes verfolgt werden kann. Die Bestimmung des NCO-Gehaltes erfolgt vorzugsweise titrimetrisch nach DIN EN ISO 11909:2007-05.

Das erfindungsgemäße Verfahren kann lösemittelfrei, aber auch in Gegenwart eines Lösemittels C durchgeführt werden. Geeignete Lösemittel sind solche, die sich gegenüber den reaktiven Gruppen der Ausgangskomponenten inert zeigen. Geeignete Lösemittel sind beispielsweise die an sich bekannten üblichen Lacklösemittel, wie z. B. Ethylacetat, Butylacetat, Ethylenglykolmonomethyl-oder -ethyletheracetat, 1-Methoxypropyl-2-acetat, 3-Methoxy-n-butylacetat, Aceton, 2-Butanon, 4-Methyl-2-pentanon, Cyclohexanon, Toluol, Xylol, Chlorbenzol, Testbenzin, höher substituierte Aromaten, wie sie beispielsweise unter den Bezeichnungen Solventnaphtha, Solvesso®, Isopar®, Nappar® (Deutsche EXXON CHEMICAL GmbH, Köln, DE) und Shellsol® (Deutsche Shell Chemie GmbH, Eschborn, DE) im Handel sind, Kohlensäureester, wie Dimethylcarbonat, Diethylcarbonat, 1,2-Ethylencarbonat und 1,2-Propylencarbonat, Lactone, wie β-Propiolacton, γ-Butyrolacton, ε-Caprolacton und ε-Methylcaprolacton, aber auch Lösemittel wie Propylenglykoldiacetat, Diethylenglykoldimethylether, Dipropylenglykoldimethylether, Butylglykolacetat, Butyldiglykolacetat, 1,3-Dioxolan, N-Methylpyrrolidon und N-Methylcaprolactam, oder beliebige Gemische solcher Lösemittel. Das Lösemittel C kann dabei einem oder beiden Reaktionspartner, der Ausgangskomponente A und/oder der Verbindung B, zugegeben werden. Das Lösemittel C kann zu jedem beliebigen Zeitpunkt während des Zudosierens der Reaktionspartner oder auch im Anschluss daran, bevorzugt aber zu Beginn der Dosierung dem Gemisch zugegeben werden.

Die erfindungsgemäßen Polyadditionsverbindungen stellen wertvolle Ausgangskomponenten für Polyurethan-Kunststoffe dar, deren Aushärtung bei Temperaturen von 100 bis 220 °C, vorzugsweise bei 120 bis 200 °C, besonders bevorzugt bei 140 bis 190 °C erfolgt.

Bevorzugt wird zur Beschleunigung der Aushärtung ein Katalysator zugegeben. Geeignete Katalysatoren sind Metallsalze und -chelate, Salze von Übergangsmetallen oder Halbmetallen, Säuren oder Basen. Als Säuren können vorzugsweise aromatische Sulfonsäuren, wie Dodecylbenzolsulfonsäure, para-Toluolsulfonsäure, Trifluormethan-sulfonsäure und Dinonylnaphthalinsulfonsäure sein, oder Schwefelsäure, Essigsäure, Trifluoressigsäure, oder Dibutylphosphat eingesetzt werden. Als Basen können vorzugsweise N-substituierte Amidine, wie 1,5-Diazabicyclo[4.3.0]non-5-en (DBN) und 1,5-Diazabicyclo[5.4.0]undec-7-en (DBU) eingesetzt werden. Als katalytisch wirkende Metallsalze und -chelate können vorzugsweise Eisen(III)-chlorid, Aluminium-tri(ethylacetoacetat), Zinkchlorid, Zink(II)-n-octanoat, Zink(II)-2-ethyl-1-hexanoat, Zink(II)-2-ethylcaproat, Zink(II)-stearat, Zink(II)-naphthenat, Zink(II)-acetylacetonat, Zinn(II)-n-octanoat, Zinn(II)-2-ethyl-1-hexanoat, Zinn(II)-ethylcaproat, Zinn(II)-laurat, Zinn(II)-palmitat, Dibutyl-zinn(IV)-oxid, Dibutylzinn(IV)-dichlorid, Dibutylzinn(IV)-diacetat, Dibutylzinn(IV)-dimaleat, Dibutylzinn(IV)-dilaurat, Dioctylzinn(IV)-diacetat, Molybdänglykolat sowie Tetraisopropyltitanat, Tetrabutyltitanat, Titan(IV)acetylacetonat, Aluminium-tri-sec-butylat, Aluminiumacetylacetonat, Aluminiumtriflat oder Zinntriflat oder beliebige Gemische solcher Katalysatoren verwendet werden.

Besonders bevorzugt werden Zinnchlorid und Zinkchlorid verwendet. Ganz besonders bevorzugt werden die zu verwendenden Katalysatoren in einem Lösemittel gelöst. Besonders bevorzugte Katalysatorlösemittel, sind beispielsweise Alkohole, wie z. B. Ethanol oder Isopropanol, die gegebenenfalls auch co-katalytisch wirken können.

Diese Katalysatoren kommen beim erfindungsgemäßen Verfahren, falls überhaupt, in einer Menge von 0,001 bis 5 Gew.-%, vorzugsweise 2,5 bis 3,5 Gew.-%, bezogen auf das Gesamtgewicht der Polyadditionsverbindung, zum Einsatz.

Als Untergründe für die mit Hilfe der erfindungsgemäßen Polyadditionsverbindungen formulierten Beschichtungen kommen beliebige, unter den Aushärtebedingungen temperaturstabile Substrate in Betracht, wie z. B. Metall, Holz, Glas, Stein, keramische Materialien, Beton, harte und flexible Kunststoffe, Textilien und Leder, die vor der Beschichtung gegebenenfalls auch mit üblichen Grundierungen versehen werden können.

Die nachfolgenden Beispiele dienen zur Verdeutlichung der vorliegenden Erfindung, sollen aber keinesfalls als Einschränkung des Schutzbereichs verstanden werden.

### Beispiele

Alle Prozentangaben beziehen sich, soweit nichts Anderslautendes vermerkt, auf das Gewicht. Die Bestimmung der NCO-Gehalte erfolgte titrimetrisch nach DIN EN ISO 11909:2007-05. Sämtliche Viskositätsmessungen erfolgten mit einem Physica MCR 51 bzw. einem Rheloab QC Rheometer der Fa. Anton Paar Germany GmbH (DE) nach DIN EN ISO 3219:1994-10 bei einer Scherrate von 250 s-1.

Die Messung der Farbzahl erfolgte spektrofotometrisch nach DIN EN ISO 6271-2:2005-03 mit einem LICO 690 Spektralcolorimeter der Fa. Lange, DE.

Die Lösemittel- und Wasserbeständigkeiten wurden nach DIN EN ISO 4628-1:2016-07 ermittelt.

Die Messung des nicht-flüchtigen Anteils (NFA) erfolgt nach DIN EN ISO 3251.

### Liste der Handelsnamen und Abkürzungen

### Oxazolin-Komponente A:

### 2,4-Dimethyl-4-hydroxymethyl-2-oxazolin hergestellt aus 2-Amino-2-methyl-1,3-propandiol und Essigsäure.

| | |
|---|---|
| Äquivalentsgewicht | 129 g/mol |
| OHZ | 434,3 mg KOH/g |

### Isocyanatkomponenten B

### Desmodur XP 2860 (Covestro Deutschland AG)

Allophanatgruppen enthaltendes Polyisocyanat auf Basis HDI, Lieferform 100 %

| | |
|---|---|
| NCO-Gehalt: | 20,0 % |
| Viskosität | 500 mPas@ 23°C |
| monomeres HDI: | 0,1 % |

### Desmodur N 3600

Aliphatisches Polyisocyanat (HDI-Isocyanurat), Lieferform 100 %

| | |
|---|---|
| NCO-Gehalt: | 23,0 % |
| Viskosität | 1200 mPas@ 23°C |
| monomeres HDI: | <0,25 % |

### Desmodur E 2863 XP

Estergruppen enthaltendesPrepolymer auf Basis HDI, Lieferform 100%

| | |
|---|---|
| NCO-Gehalt: | 11,0% |
| Viskosität | 1350 mPas@ 23°C |
| monomeres HDI: | <0,3 % |

- Essigsäure (99,8%) wurde von der Firma Acros Organics, Schwerte, Deutschland bezogen.
- 2-Amino-2-methyl-1,3-propandiol wurde von der Firma Merck (Sigma Aldrich) bezogen.
- Zinkchlorid wurde von der Firma Merck (Sigma Aldrich) bezogen.
- Zinnchlorid wurde von der Firma Merck (Sigma Aldrich) bezogen.
- Lösemittel wie Butylacetat (BA) und Methoxypropylacetat (MPA) wurden von der Firma Azelis bezogen.

### Erfindungsgemäße Beispiele 1 bis 4:

Die jeweilige Isocyanat-Komponente wird in der entsprechenden Menge Lösemittel vorgelegt und auf 60 °C aufgeheizt. Bei einer Temperatur von 60 °C wird 2,4-Dimethyl-4-hydroxymethyl-2-oxazolin über einen Zeitraum von 30 Minuten zu dosiert. Das Reaktionsgemisch wird bei 60 °C gerührt bis der theoretische NCO Gehalt von 0,0 Gew.-% erreicht ist.

Nach Abkühlen auf Raumtemperatur werden die Kenndaten NCO-Gehalt, Viskosiät und Farbzahl ermittelt.

**Tabelle 1: Übersicht erfindungsgemäße Beispiele und ermittelte Kenndaten**

| | **Beispiel 1** | **Beispiel 2** | **Beispiel 3** | **Beispiel 4** |
|---|---|---|---|---|
| | [g] | [g] | [g] | [g] |
| Desmodur N 3600 | 88,33 | | | 123,67 |
| Desmodur XP 2860 | | 131,28 | | |
| Desmodur E 2863 XP | | | 157,57 | |
| 2,4-Dimethyl-4-hydroxymethyl-2-oxazolin | 63,26 | 79,37 | 54,28 | 86,33 |
| Butylacetat | | 90 | 90 | 90 |
| Methoxypropylacetat | 64,2 | | | |

| **Kenndaten** | | | | |
|---|---|---|---|---|
| NCO-Gehalt | 0,00 w% | 0,02 w% | 0,00 w% | 0,03 w% |
| Viskosität @23°C | 11400 mPas | 1250 mPas | 975 mPas | 5080 mPas |
| Farbzahl | 19 Hz | 13 Hz | 24 Hz | 13 Hz |
| NFA | 68,7% | 69,3% | 67,3% | 70,0% |

### Lackprüfungen

Alle Prozentangaben beziehen sich, soweit nichts anderes angegeben wird, auf das Gewicht. Alle Versuche wurden bei 23 °C und 50% relativer Feuchtigkeit durchgeführt.

Für den Test der Lösemittelbeständigkeiten wurden die Lösemittel Xylol (folgend auch als "Xy" abgekürzt), Methoxypropylacetat (folgend auch als "MPA" abgekürzt), Ethylacetat (folgend auch als "EA" abgekürzt) und Aceton (folgend auch als "Ac" abgekürzt) verwendet. Die Kontaktzeit betrug 1 min bzw. 5 min. Für die Messung der Wasserbeständigkeiten betrug die Kontaktzeit jeweils 1 h. Die Abmusterung wurde entsprechend der angeführten Norm durchgeführt. Die Prüffläche wurde visuell und durch Verkratzung beurteilt, dabei wurde folgende Klassifikation vorgenommen: 0 = Keine Veränderung feststellbar; 1 = Quellungsring, Oberfläche hart, nur sichtbare Veränderung; 2 = Quellungsring, geringe Erweichung; 3 = deutliche Erweichung (evtl. geringe Blasenbildung); 4 = Starke Erweichung (evtl. starke Blasenbildung), durchritzbar bis zum Untergrund; 5 = Beschichtung komplett zerstört ohne Fremdeinwirkung.

Die Bestimmung der Pendeldämpfung nach König erfolgte nach DIN EN ISO 1522:2007-04 auf Glasplatten.

### Anwendungsbeispiele (erfindungsgemäß)

Die folgende Tabelle 2 zeigt unterschiedlich katalysierte und nicht-katalysierte Lackformulierungen auf Basis der erfindungsgemäßen Polyadditionsverbindungen.

**Tabelle 2: Zusammensetzungen der Lackmischungen.**

| Produkt | | Lack 1 (Vgl ) | Lack 2 (Vgl ) | Lack 3 (Vgl ) | Lack 4 (Vgl ) | Lac k 5 | Lac k 6 | Lac k 7 | Lac k 8 | Lac k 9 | Lac k 10 | Lac k 11 | Lac k 12 |
|---|---|---|---|---|---|---|---|---|---|---|---|---|---|
| | NFA [%] | [g] | [g] | [g] | [g] | [g] | [g] | [g] | [g] | [g] | [g] | | |
| Beispiel 1 | 68,7 % | 10 | | | | 10 | | | | 10 | | | |
| Beispiel 2 | 67,3 % | | 10 | | | | 10 | | | | 10 | | |
| Beispiel 3 | 70,0 % | | | 10 | | | | 10 | | | | 10 | |
| Beispiel 4 | 69,3 % | | | | 10 | | | | 10 | | | | 10 |
| Zinkchlo rid (in Ethanol) | 10,0 % | | | | | 2,0 6 | 2,0 2 | 2,1 0 | 2,0 8 | | | | |
| Zinnchlo rid (in Ethanol) | 10,0 % | | | | | | | | | 2,0 6 | 2,0 2 | 2,1 0 | 2,0 8 |
| Butylace tat | | | | | | 3 | 3 | 3 | 3 | 3 | 3 | 3 | 3 |

Diese Lackformulierungen wurden mit einem Rakel auf Glasplatten aufgezogen und jeweils 30 min bei 140, 160 und 190°C eingebrannt. Die Schichtdicke betrug in allen Fällen 35-40 µm.

Im nächsten Schritt wurde die Lösemittel- und Wasserbeständigkeit der erhaltenen Beschichtungen in Abhängigkeit von der Art der verwendeten Katalysatoren und der Einbrennbedingungen untersucht (Tabelle 3). Die hier gezeigten Versuche zeigen deutlich, dass die verwendeten Katalysatoren zu unterschiedlichen Beständigkeiten führen.

Die Lacksysteme 5, 6, 8, 9, 10, 11 und 12 erzielen sehr gute Lösemittelbeständigkeiten nach Einbrennen bei 190 °C. Nach Einbrennen bei 160 °C führen vor allem Lack 9, 10 und 12 zu sehr guten Lösemittelbeständigkeiten. Lacksystem 7 und 11 führen zu deutlich schlechteren Beständigkeiten. Eine Einbrenntemperatur von 140 °C führt bei allen Systemen zur Abnahme der Lösemittelbeständigkeit.

Für Lacksysteme die ohne Zugabe von Katalysator untersucht wurden, kann auch bei einer Einbrenntemperatur von 190 °C keine ausreichende Vernetzung beobachtet werden. Die Formulierung führt zu klebrigen bzw. zu sehr weichen und wenig lösemittelbeständigen Filmen.

**Tabelle 3: Lösemittel- und Wasserbeständigkeiten unter verschiedenen Einbrennbedingungen: 30 min, 140 - 190 °C**

| | Lac k 1 (Vg 1) | Lac k 2 (Vg 1) | Lac k 3 (Vg 1) | Lac k4 (Vg 1) | Lack 5 | Lac k 6 | Lac k 7 | Lac k 8 | Lac k9 | Lac k 10 | Lac k 11 | Lac k 12 |
|---|---|---|---|---|---|---|---|---|---|---|---|---|
| **30** min, 190 **°C** | | | | | | | | | | | | |
| Xy/MP A/EA/ Ac n. 1d (1 min) | 555 5 | n.b. | n.b. | 555 5 | 0000 | 001 3 | 212 1 | 000 0 | 000 0 | 000 2 | 000 0 | 000 0 |
| Xy/MPA/EA/Ac n. 1d (5 min) | | | | | 0000 | 002 4 | 333 3 | 000 1 | 000 0 | 000 0 | 000 0 | 000 0 |
| Wasserbeständig keit | | | | | 0 | 0 | 3 | 0 | 3 | 3 | 3 | 3 |

| **30 min, 160** °C | | | | | | | | | | | | |
|---|---|---|---|---|---|---|---|---|---|---|---|---|
| Xy/MPA/EA/Ac n. 1d (1 min) | | | | | 2355 | 555 5 | - | 135 5 | 000 0 | 000 2 | 223 3 | 000 0 |
| Xy/MPA/EA/Ac n. 1d (5 min) | | | | | 3555 | - | | 255 5 | 000 0 | 000 3 | 333 3 | 000 1 |
| Wasserbeständig keit | | | | | 4 | | | 4 | 5 | 4 | 5 | 5 |

| **30 min, 140 °C** | | | | | | | | | | | | |
|---|---|---|---|---|---|---|---|---|---|---|---|---|
| Xy/MPA/EA/Ac n. 1d (1 min) | | | | | 5555 5 | - | - | 455 5 | 002 4 | 113 5 | 334 4 | 002 5 |
| Xy/MPA/EA/Ac n. 1d (5 min) | | | | | - | | | - | 013 5 | 224 5 | 444 5 | 002 5 |
| Wasserbeständig keit | | | | | 5 | | | 5 | 5 | 5 | 5 | 5 |

Wie Tabelle 4 zu entnehmen ist, führen Lack 7 und 11 zu sehr weichen Filmen. Die restlichen Ergebnisse zeigen, dass die Pendelhärten bei einer Einbrenntemperatur von 190 °C für die Lacksysteme zwischen 185 und 215 Pendelsekunden liegen. Bei einer Einbrenntemperatur von 160 °C liegen die Pendelhärten zwischen 150 und 215 Pendelsekunden. Bei einer Einbrenntemperatur von 140 °C liegen die Pendelhärten zwischen 80 und 185 Pendelsekunden.

**Tabelle 4: Dämpfungsdauer nach König.**

| | Lack 1 | Lack 2 | Lack 3 | Lac k 4 | Lac k 5 | Lac k 6 | Lac k7 | Lac k8 | Lack 9 | Lack 10 | Lack 11 | Lack 12 |
|---|---|---|---|---|---|---|---|---|---|---|---|---|
| **30 min, 190 °C** | 50 | klebr ig | klebr ig | 53 | 215 | 195 | 15 | 205 | 185 | 190 | 40 | 210 |
| **30 min, 160 °C** | | | | | 185 | 50 | - | 150 | 200 | 205 | 40 | 215 |
| **30 min, 140 °C** | | | | | 140 | - | - | 80 | 185 | 95 | 15 | 155 |

## Patentansprüche

1. Polyadditionsverbindung enthaltend mindestens ein Strukturelement der allgemeinen Formel 1 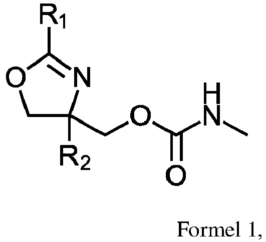 in welcher die Reste R₁ und R₂ für gleiche oder verschiedene Reste stehen und gegenüber Isocyanatgruppen inerte, lineare oder verzweigte aliphatische, cycloaliphatische, gegebenenfalls substituierte aromatische oder araliphatische Reste mit jeweils bis zu 18 Kohlenstoffatomen stehen, die bis zu drei oder vier Heteroatome enthalten können und in Kombination untereinander zusammen einen Ring bilden können.

2. Verfahren zur Herstellung einer Polyadditionsverbindung enthaltend mindestens ein Strukturelement der allgemeinen Formel 1 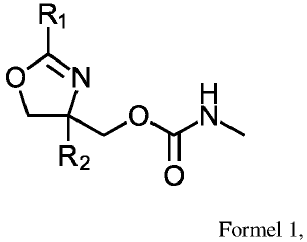 in welcher die Reste R₁ und R₂ für gleiche oder verschiedene Reste stehen und gegenüber Isocyanatgruppen inerte, lineare oder verzweigte aliphatische, cycloaliphatische, gegebenenfalls substituierte aromatische oder araliphatische Reste mit jeweils bis zu 18 Kohlenstoffatomen stehen, die bis zu drei oder vier Heteroatome enthalten können und in Kombination untereinander zusammen einen Ring bilden können,
durch Umsetzung einer Oxazolinkomponente (A) der allgemeinen Formel 2 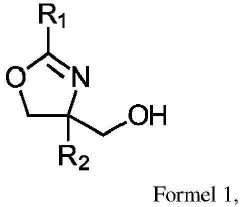 in welcher R₁ und R₂ die oben genannte Bedeutung haben, mit einer Isocyanatkomponente (B) enthaltend mindestens eine cycloaliphatische, aliphatische, aromatische und/oder araliphatische gebundene Isocyanatgruppe.

3. Polyadditionsverbindung gemäß Anspruch 1 oder Verfahren zur Herstellung einer Polyadditionsverbindung gemäß Anspruch 2, **dadurch gekennzeichnet, dass** R₁ und R₂ für gleiche oder verschiedene, gegenüber Isocyanatgruppen inerte, lineare oder verzweigte aliphatische oder cycloaliphatische Reste mit bis zu 18 Kohlenstoffatomen stehen.

4. Polyadditionsverbindung oder Verfahren zur Herstellung einer Polyadditionsverbindung gemäß Anspruch 3, **dadurch gekennzeichnet, dass** R₁ und R₂ für gleiche oder verschiedene, gegenüber Isocyanatgruppen inerte, lineare aliphatische Reste mit bis zu drei Kohlenstoffatomen stehen.

5. Polyadditionsverbindung oder Verfahren zur Herstellung einer Polyadditionsverbindung gemäß Anspruch 4, **dadurch gekennzeichnet, dass** R₁ und R₂ für jeweils eine CH₃-Gruppe stehen.

6. Verfahren gemäß einem der Ansprüche 2 bis 5, **dadurch gekennzeichnet, dass** als Isocyanatkomponente B beliebige cycloaliphatische, aliphatische, aromatische und/oder araliphatische Diisocyanate oder durch Modifizierung von beliebigen, monomeren cycloaliphatischen, aliphatischen, aromatischen und/oder araliphatischen Diisocyanaten erhältliche Di-und/oder Polyisocyanate oder Isocyanat-funktionelle Prepolymere eingesetzt werden.

7. Verfahren gemäß Anspruch 6, **dadurch gekennzeichnet, dass** als Isocyanatkomponente B durch Modifizierung von monomeren cycloaliphatischen, aliphatischen, aromatischen und/oder araliphatischen Diisocyanaten erhältliche Di- und/oder Polyisocyanate mit Uretdion-, Isocyanurat-, Allophanat-, Biuret-, Iminooxadiazindion- und/oder Oxadiazintrionstruktur zum Einsatz kommen.

8. Verfahren gemäß Anspruch 6 oder 7, **dadurch gekennzeichnet, dass** die Isocyanatkomponente B ausschließlich aliphatisch und/oder cycloaliphatisch gebundene Isocyanatgruppen aufweist, und/oder ausschließlich Isocyanurat- und/oder Allophanatstrukturen aufweist.

9. Verfahren gemäß einem der Ansprüche 6 bis 8, **dadurch gekennzeichnet, dass** als die Isocyanatkomponente B eine hydrophile Modifikation aufweist.

10. Verwendung einer Polyadditionsverbindung gemäß einem der Ansprüche 1 bis 5, gegebenenfalls in Kombination mit katalytisch-wirkenden Metallsalzen und/oder -chelaten, zur Herstellung von Polyurethan Kunststoffen.

11. Beschichtungsmittel enthaltend eine Polyadditionsverbindung gemäß einem der Ansprüche 1 bis 5 und gegebenenfalls katalytisch-wirkende Metallsalze und/oder -chelate.

12. Verwendung gemäß Anspruch 10 oder Beschichtungsmittel gemäß Anspruch 11, wobei katalytisch-wirkende Metallsalze und/oder -chelate vorhanden sind und diese in einem alkoholischen Lösemittel gelöst sind.

13. Substrat beschichtet mit einem Beschichtungsmittel gemäß Anspruch 11 oder 12.
